# EUROPEAN PATENT APPLICATION

(11) **EP 2 110 128 A1**
(43) Date of publication of application: **21.10.2009**
(21) Application number: 07822948.1
(22) Date of filing: 24.09.2007
(51) Int. Cl.: A61K 31/095, A23K 1/17

(54) **USE OF PARTICULAR ANTIBACTERIAL COMPOUNDS, WHICH ARE DERIVED FROM ALLIACEAE, AS NATURAL ADDITIVES IN ANIMAL FEED**

(30) Priority: 27.09.2006 ES 200602446
(71) Applicant: DMC Research Center, S.L., 18620 Alhendin Granada (ES)
(72) Inventor: GARCIA PAREJA, Mª Pilar, 18620 Alhendin (Granada) (ES); LARA CAMBIL, Armando, 18620 Alhendin (Granada) (ES); RUBIO SANMILLÁN, Luis A., 18620 Alhendin (Granada) (ES); MOLINA ALCAIDE, Eduarda, 18620 Alhendin (Granada) (ES)
(74) Representative: Munoz Garcia, Antonio
(86) International application number: PCT/ES2007/000541
(87) International publication number: WO 2008/037827

(57) **Abstract**

The invention relates to the use of particular antibacterial compounds, which are derived from alliaceae, as natural additives in animal feed, through their inclusion in the diet, for the prevention and/or treatment of digestive pathological processes without affecting the beneficial microbiota, said compounds being the following:
- propyl propylthiosulfinate
- n-butyl n-butylthiosulfinate
- propyl propylthiosulfonate
- a compound consisting of a mixture of E and Z isomers or of each of them separately: (E- and Z-4,5,9-trithiadodeca-1,6,11-triene-9-oxide)
- 3-vinyl-4-H-1,2-dithiin
- 2-vinyl-4-H-1,3-dithiin

which can be used separately as the only pure active ingredients (purity greater than 95%), as combinations of several of them or in mixtures with other synergistic products; they may be in encapsulated structures, supported on different inert materials or food coatings forming part of the animal diet. The concentrations can vary between: 1-5 ppm at least and 10% (w/w) at most, according to the compound, support, their combination, type of diet, condition, etc.

## Description

### Object of the Invention

As expressed by the title of this specification, the invention relates to the use of particular antibacterial compounds, which are derived from Alliaceae, as natural additives in animal feed.

More specifically, the object of the invention consists of the use of particular compounds derived from the *Allium* genus as antimicrobial agents in animal feed, and as an alternative, due to their antibacterial nature, to the use of antibiotics as growth promoters.

### Field of Application

The field of application of the present invention is the animal feed industry.

### Background of the Invention

The European Union has drastically reduced the use of antibiotics in animal feed as growth promoters, having been prohibited in January 2006.

The alternative herein proposed for these additives against the use of antibiotics in feed as growth promoters in animal feed, is actually valid as is shown with the obtained and previously summarized results.

The proposed compounds favor the defensive capability of the animal against digestive pathological processes.

Besides promoting animal growth (by favoring the reduction of diet nutrient degradability and developing a control of infections and/or of microbial imbalances), they have the advantage of being compounds that are traditionally present in food (garlic, onion, leeks, etc.).

Therefore, said additives would be a harmless alternative which in turn prevent the consumption of antibiotics in food and, therefore, the subsequent occurrence of resistance to antibiotics in consumers.

On the other hand, these compounds contribute to reducing the greenhouse effect derived from the release of gas produced during an ineffective food digestion process in ruminant animals.

This effect is very important today in ruminant animal nutrition because the gas that is released as a result of the fermentation of foods is essentially methane, a huge greenhouse effect gas.

They can further contribute to reducing the elimination of nitrogen into the medium through animal excreta.

It should be mentioned that the applicant knows of several references in which there are studies on the same compounds mentioned in the present invention, however, they do so from the view point of descriptive chemistry, antimicrobial capacity in general or use in human health, but not in animal feed, as in the case at hand.

References to garlic derivatives (essential garlic oil, garlic powder, allicin, alliin, allyl disulfide,...) as components of food supplements to improve animal nutrition are also known, but no specific compounds are mentioned.

Likewise, patent WO 2005/089571 relating to the use of extracts and compounds of Allium-genus plants as preservatives in the food and agri-food industries should be mentioned, which patent, however, only provides a possible use of said compounds with no basis and without providing any efficacy results in animal feed with diets including said compounds.

Other patents, such as patent US 5,705,152, which claims alliin and alliinase compounds to form allicin and therefore involves compounds different from those herein described, or patent KR 20020048347 relating to a method for obtaining Allium tuberosum juices and extract, also involve different compounds.

It can therefore be indicated that although the applicant knows of the existence of several documents relating to the use of garlic or the derivatives thereof in animal feed, no direct references have been found which mention the use of the compounds herein described in animal feed or as an alternative to the use of growth promoting antibiotics as provided in the present invention, being able to assert that the applicant is unaware of the existence of any other patent which has features similar to those herein described.

### Description of the Invention

Thus the invention basically consists of the use of particular compounds or specific compounds present in *Alliaceas* plants with a proven antimicrobial capacity.

Said compounds have a broad spectrum of antibacterial and antifungal activity, with a high inhibitory potency at low doses.

These compounds and the derivatives thereof are proposed as a valid and effective alternative to the use of antibiotics as growth promoters in animal feed (ruminant and monogastric animals).

The effectiveness of the compounds the use of which is proposed is due, at least in part, to its high antibacterial potency, which causes effective control of the potentially harmful intestinal microbiota, therefore preventing digestive pathological processes.

Gastrointestinal infections are extremely relevant for example in young animals.

A specific case is that of the problems derived from diarrhea in piglets, as they are processes with a very high impact from both the sanitary and production points of view.

Thus by favoring the health of the animal its well-being, growth and production output are favored.

On the other hand, including these compounds in rations for ruminant animals reduces the release of fermentation gases, mainly methane, a gas that is directly involved in the so-called "greenhouse effect".

In other words, the use of the additive would have a positive environmental impact as it would contribute to reducing the greenhouse effect.

Additionally, including the additive in feed for ruminant animals may have a positive effect on the medium through reducing protein degradation in the rumen, which would reduce the elimination of nitrogen in excreta (animal urine and feces) and, therefore, environmental pollution with nitrogenous products.

The use of these additives in diets for animals for production would provide a valid alternative to the use of antibiotics as growth promoters.

This is particularly relevant today given that the use of antibiotics as growth promoters has recently been prohibited in European Union countries.

Therefore the following specific compounds are proposed as additives alternative to the use of antibiotics in feed, also as animal growth promoters:
- propyl propylthiosulfinate
- n-butyl n-butylthiosulfinate
- propyl propylthiosulfonate
- 3-vinyl-4-H-1,2-dithiin
- 2-vinyl-4-H-1,3-dithiin
- A compound consisting of a mixture of E and Z isomers or of each of them separately: (E- and Z-4,5,9,-trithiadodeca-1,6,11-triene-9-oxide)

Said compounds can be used both as the only additives in the diet (i.e., separately, with a purity greater than 95%) or as combinations of some of them or with other allowed synergistic products. Said compounds can also be encapsulated or supported on different inert materials or food coatings forming part of the animal diet.

The doses of the active compounds in the final formulation of the diet in which they are included as microbial growth promoters may cover the range:
- Minimum dose of 5 ppm
- Maximum dose: of 5% (w/w), expressed in pure active ingredients (purity > 95%, determined by HPLC (high performance liquid chromatography), or up to 10% in the case of combinations of several of these compounds.

The expiration period for the products or approximate interval will be 6 months- 1 year, depending on the type of product and baseline diet in which they are included.

The additives are acceptable for consumers of animal products (meat and milk) having a beneficial effect on the production and health condition of the animals, the quality of their products and the environment.

A number of different assays have been performed to demonstrate the effectiveness of the products proposed as additives, being able to provide a considerable amount data of such additives both in tables and graphs.

The obtained data relate to: 1) the effect on the digestive microbiota of monogastric animals and ruminant animals; 2) the dose-response relation; 3) the study of the microbial groups involved and evolution thereof depending on the specific product used; 4) the increase of the efficiency of ruminal processes; 5) the improvement in recovering nutrients from the diet; 6) the control of microorganisms causing undesirable fermentations; 7) the determination of the most effective doses to achieve the beneficial effects sought (favoring growth), palliating the harmful effects (digestive pathological processes and inefficient fermentations) through including these additives in diets.

Assays are conducted to evaluate the efficacy of the proposed antimicrobial compounds through the diet, and therefore their usefulness as animal growth promoters and in preventing and/or treating different gastrointestinal pathologies.

These assays were aimed at animal feed of both ruminant and monogastric animals.

Some results which may serve as an example regarding the high antibacterial capacity of the compounds which are proposed against the main microbial groups involved in some of the most common digestive pathological processes are shown.

In addition to these results regarding the antimicrobial capacity, other results relating to the promoting capacity of the proposed additives through the animal diet are also explained.

Said assays were conducted in ruminant animal and monogastric animal models in the Animal Nutrition Unit of the Experimental Station of Zaidin (CSIC, Granada).

### Description of the Drawings

To complement the description being made and for the purpose of aiding to better understand the features of the invention, a set of drawings is attached hereto as an integral part thereof showing the following:

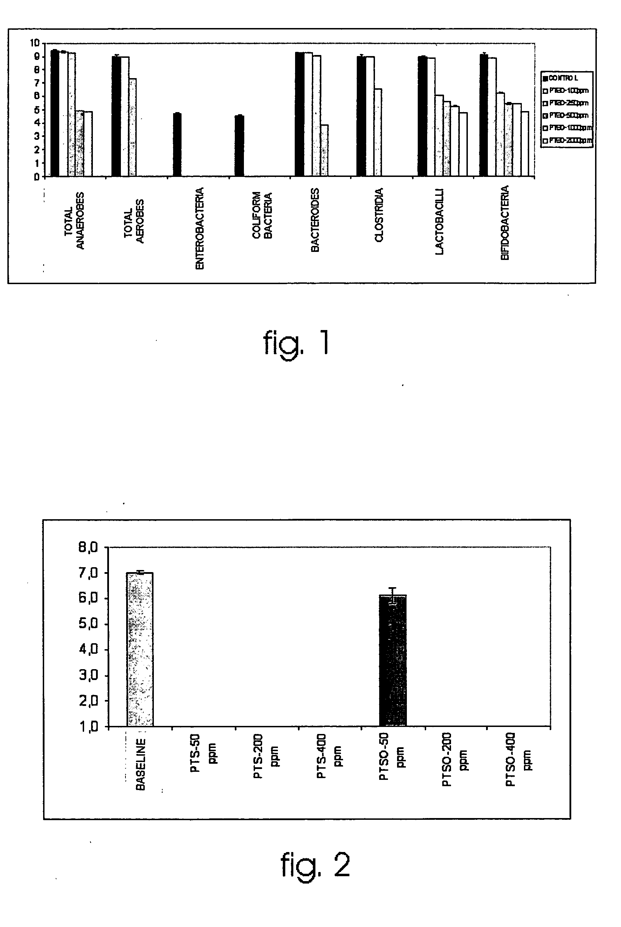

Figure 1 is a graph relating to Example 1 (study of the porcine digestive microbial composition) showing the effect of different concentrations of the compound PTSO after 24 hours of *in vitro* fermentation on the total anaerobic bacteria, total aerobic bacteria, enterobacteria, coliform bacteria, bacteroides, clostridia, lactobacilli and bifidobacteria count present in pig feces. The vertical axis of the graph determines the log10(cfu/g feces) and the horizontal axis determines the bacterial group.
Figure 2 is a graph relating to Example 2 and shows the effect of the addition of different concentrations of the compounds PTS and PTSO after 24 hours of *in vitro* fermentation on the enterobacteria count in pig feces. The mean values affected by different indexes indicate significant differences (p < 0.05). The vertical axis of the graph determines the log10(cfu/g feces) and the horizontal axis determines the compound.

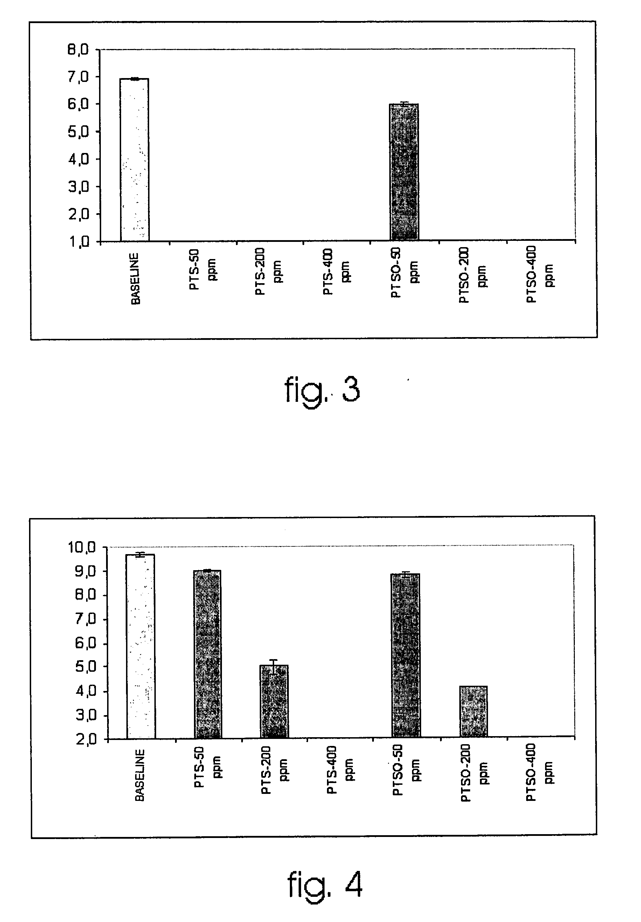

Figure 3 is a graph also relating to Example 2 and shows the effect of the addition of different concentrations of the compounds PTS and PTSO after 24 hours of *in vitro* fermentation on the coliform bacteria count in pig feces. The mean values affected by different indexes indicate significant differences (p < 0.05). The vertical axis of the graph determines the log10(cfu/g feces) and the horizontal axis determines the compound.
Figure 4 is a graph relating to Example 2 and shows the effect of the addition of different concentrations of the compounds PTS and PTSO after 24 hours of *in vitro* fermentation on the bacteroides count in pig feces. The mean values affected by different indexes indicate significant differences (p < 0.05). The vertical axis of the graph determines the long10(5 cfu/g feces) and the horizontal axis determines the compound.

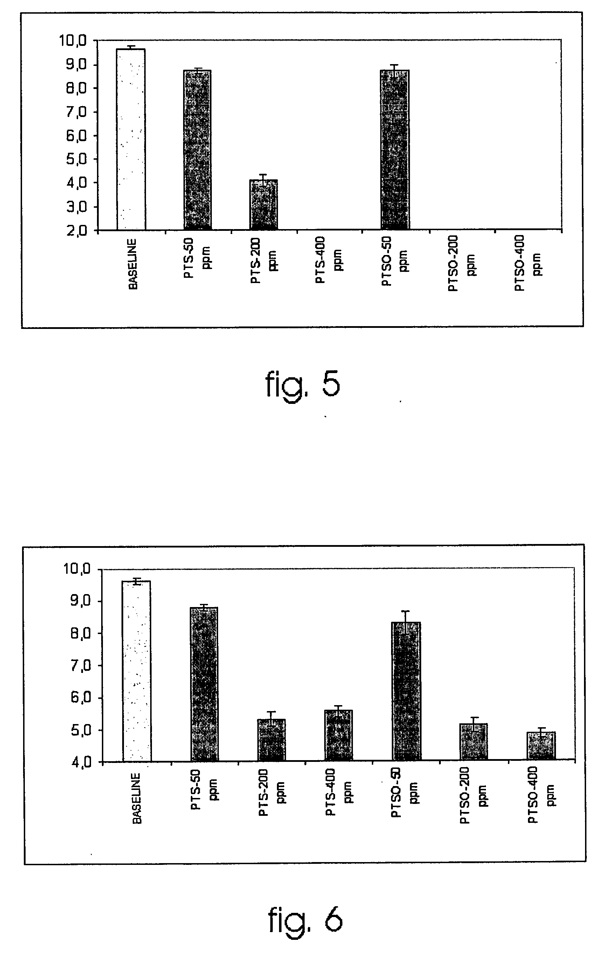

Figure 5 is a graph relating to Example 2 and shows the effect of the addition of different concentrations of the compounds PTS and PTSO after 24 hours of *in vitro* fermentation on the clostridia count in pig feces. The mean values affected by different indexes indicate significant differences (p < 0.05). The vertical axis of the graph determines the log10(cfu/g feces) and the horizontal axis determines the compound.
Figure 6 is a graph relating to Example 2 and shows the effect of the addition of different concentrations of the compounds PTS and PTSO after 24 hours of *in vitro* fermentation on the lactobacilli count in pig feces. The mean values affected by different indexes indicate significant differences (p < 0.05). The vertical axis of the graph determines the log10(cfu/g feces) and the horizontal axis determines the compound.

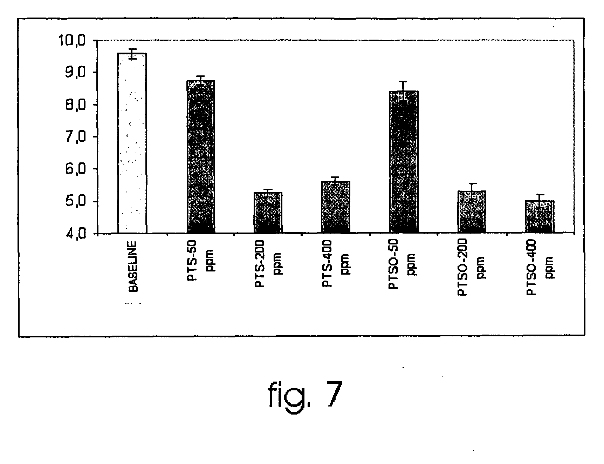

Figure 7 is a graph relating to Example 2 and shows the effect of the addition of different concentrations of the compounds PTS and PTSO after 24 hours of *in vitro* fermentation on the bifidobacteria count in pig feces. The mean values affected by different indexes indicate significant differences (p < 0.05). The vertical axis of the graph determines the log10(cfu/g feces) and the horizontal axis determines the compound.

### Preferred Embodiment of the Invention

Some assays conducted to demonstrate the efficacy of the compounds proposed as additives in the diets of monogastric animals are described below.

The abbreviations PTS and PTSO have been used throughout all the examples as abbreviations for the following compounds:
- propyl propylthiosulfinate, referenced with the abbreviation PTS
- propyl propylthiosulfonate, referenced with the abbreviation PTSO.

A.- Examples 1 and 2 to demonstrate the efficacy of the compounds proposed as additives in the diets of monogastric animals

### Example 1: Study conducted on the porcine digestive microbial composition.

The number of microorganisms forming the gastrointestinal microbiota in this animal species is very high (>1014 cfu/g) and includes bacteria representative of quite varied microbial groups (enterobacteria in general and coliform bacteria in particular, bacteroides, clostridia, bifidobacteria, lactobacilli, etc., as well as many other aerobic and anaerobic bacteria species), between 400-500 different species having been identified (see for example Vanbelle et al., 1990).

The effect of the compounds on the composition of the intestinal microbiota of pigs in the post-weaning stage has been studied.

The dose-effect relation and the optimal dose for the control of harmful microorganisms (enterobacteria, clostridia) without affecting the potentially beneficial microbiota such as bifidobacteria and lactobacilli have been determined.

The results obtained from the described assays and from others conducted prove the capacity of the proposed products for the control of the growth of potentially pathogenic microorganisms (coliform bacteria, enterobacteria, clostridia) in animal samples.

These microbial species are involved in most diarrheal and other gastrointestinal processes in piglets (Zeyner and Boldt, 2005).

The described assays are carried out using complete bacterial inocula obtained from feces of piglets in post-weaning stage.

The incorporation in the culture medium of one of the compounds (referenced as PTSO) in a range of concentrations between 50 and 2000 mg/L inhibited the growth of all the bacterial groups studied (total anaerobes, total aerobes, enterobacteria, coliform bacteria, bacteroides, clostridia, lactobacilli and bifidobacteria), although the degree of sensitivity varies depending on the microbial type and group studied (Figure 1).

These results indicate that the groups which are inhibited at lower doses are enterobacteria, coliform bacteria and clostridia, which are the microorganisms with a higher incidence in digestive pathological processes of pigs.

These additives are therefore proposed as being effective for the prevention of digestive pathological processes.

The microorganisms least affected by the additives used are bifidobacteria and lactobacilli, which are bacteria that form part of the intestinal microbiota considered beneficial, and therefore it is especially relevant data.

### Example 2:

This example shows the results of studies subsequent to those of Example 1, which confirm and increase the information.

Two of the compounds (referenced as PTS and as PTSO) are assayed in a lower dose range (50-400 mg/L).

It is demonstrated that a concentration of 50 mg/L of one of the products is able to completely suppress the growth of enterobacteria in general, and of coliform bacteria in particular (Figures 2 and 3).

The bacteroides and clostridia population (Figures 4 and 5) behaved in a similar manner in the presence of the different assayed concentrations of the compounds PTS and PTSO.

The addition of both compounds at 50 mg/L caused a slight though significant population decrease.

This decrease was more pronounced (approximately 50%) when the concentration was 200 mg/L.

The addition of any of the two compounds at 400 mg/L meant the disappearance of both microbial groups.

For their part, the lactobacilli and bifidobacteria groups (Figures 6 and 7) experienced a slight decrease after adding 50 mg/L of both compounds.

The addition of 200 mg/L of both PTS and PTSO caused a logarithmic reduction in the populations of approximately 50%.

Said decrease was maintained or slightly increased when both compounds were added at the concentration of 400 mg/L.

This result indicates a much higher resistance of these species to the effects of these products.

In summary, it can be seen that the sensitivity of the studied bacterial groups to these additives is, in decreasing order: coliform bacteria = enterobacteria > clostridia > bacteroides > bifidobacteria = lactobacilli.

Some assays conducted to prove the effect of the compounds proposed as additives in feed rations for ruminant animals are listed below.
B.- Examples 3 and 4 to prove the efficacy of the compounds proposed as additives in feed rations for ruminant animals

Example 3: The effect of different doses of the compounds object of study on the ruminal degradation of two different diets the composition of which is in Table 1 is studied.

The diets consist of alfalfa hay (standard maintenance diet for ruminant animals) or of a mixture of 50% alfalfa hay and a commercial concentrate (standard lactation diet for ruminant animals).

*In vitro* incubation is performed using ruminal fluid of goats as inoculum.

**Table 1.- Chemical composition of the two experimental diets**

| Ingredients | Diet D1 | Diet D2 |
|---|---|---|
| | Alfalfa hay | Alfalfa hay + concentrate (1:1) |
| DM, *g*/*100g Fresh matter g*/*100g Dry matter* | 92.7 | 91.8 |
| Organic matter | 90.5 | 90.2 |
| Crude protein | 16.5 | 16.6 |
| Neutral detergent fiber | 51.4 | 41.4 |
| Acid detergent fiber | 38.2 | 27.8 |
| Acid detergent lignin | 9.27 | 4.76 |

| | | |
|---|---|---|
| FM: fresh matter; DM: dry matter; D1: 100% alfalfa hay; D2: 50% alfalfa hay: 50% commercial concentrate | | |

The major difference between the two diets is based on their content in fibrous components (neutral detergent fiber, acid detergent fiber and lignin), which is higher in diet 1 than in diet 2.

The effects are shown in Tables 2 to 6 and indicate:
i) Dose of the compound PTS between 50 and 750 ppm in relation to the volume of inoculum determine a reduction of the degradation of dry matter of diet D1 and do not affect the degradation of organic matter after 24 hours of *in vitro* incubation (Table 2).
   This indicates an antimicrobial effect of the assayed compound (PTS) on the microbiota of the ruminal inoculum (ruminal fluid of goats).
   This effect is interesting insofar as the protection of the degradation of nutrients in rumen involves an increase of the amount of nutrients available for the animal in the small intestine (Salawu et al., 1999)
   There is further interest deriving from the beneficial effect that the reduction of the elimination of nitrogen in feces and urine may have on the environment.
ii) A dose of 2000 ppm of the compound called PTSO determines a higher reduction of the degradation of dry matter and organic matter of diet D1 after the *in vitro* incubation thereof for 48 hours (Table 3).
   This indicates a stronger antimicrobial effect of this dose and of this compound on the microbiota of the inoculum (ruminal fluid of goats). Such a drastic reduction of the degradability may not be beneficial.
iii) The study of the effect of doses of 10, 25, 50, 300, 750, 1000 and 2000 ppm of the compounds called PTS and PTSO (Tables 3, 4 and 5) indicates (Table 6) that the degradation of diet D1 is affected by doses greater than 25 ppm of both the compound PTS and the compound PTSO.

The degradation of diet D2, however, is affected by lower doses (10 ppm).

Therefore, the dose required to promote an effect on the degradation of a diet seems to depend on the type of diet.

Since diet D1 is a standard diet to be used with ruminant animals that are not productive and diet D2 is standard for the feed of ruminant animals that are productive, the handling of the dose and of the compound to be used as an additive will depend on the physiological condition of the animal.

In any case, the reduction of degradation in rumen indicates an antimicrobial effect which can be exerted on total anaerobes, cellulolytic bacteria (the case of type D1 diets rich in fodder), amylolytic bacteria (the case of type D2 diets which include 50% concentrate) and/or ruminal protozoa.

**Table 2.- Degradation of dry matter (DDM) and organic matter (DOM) of diet D1 after incubation for 24 hours with ruminal inoculum containing 0, 50, 300 and 750 ppm of PTS.**

| Concentration PTS, ppm | DDM, % | Mean DDM, % | DOM, % | Mean DOM, % |
|---|---|---|---|---|
| *0* | 40.2 | 40.0 | 42.0 | 38.5 |
| *0* | 33.1 | | 34.9 | |
| *0* | 41.5 | | | |
| *0* | 48.8 | | | |
| *0* | 38.3 | | | |
| *0* | 38.1 | | | |
| *50* | 37.7 | 37.1 | 38.0 | 38.5 |
| *50* | 37.9 | | 38.9 | |
| *50* | 34.1 | | | |
| *50* | 35.6 | | | |
| *50* | 37.7 | | | |
| *50* | 39.3 | | | |
| *300* | 38.5 | 37.4 | | |
| *300* | 40.4 | | | |
| *300* | 37.6 | | | |
| *300* | 36.0 | | | |
| *300* | 35.7 | | | |
| *300* | 36.2 | | | |
| *750* | 40.8 | 39.5 | 39.4 | 38.1 |
| *750* | 37.7 | | 36.8 | |
| *750* | 43.2 | | | |
| *750* | 39.5 | | | |
| *750* | 37.9 | | | |
| *750* | 38.0 | | | |

**Table 3.- Degradation of dry matter (DDM) of diet D1 after incubation for 48 hours with ruminal inoculum containing 0 and 2,000 ppm of PTS.**

| Concentration PTSO, ppm | DDM, % | Mean DDM, % |
|---|---|---|
| *0* | 51.1 | 49.7 |
| *0* | 49.8 | |
| *0* | 49.3 | |
| *0* | 50.0 | |
| *0* | 48.3 | |
| *0* | 49.6 | |
| *2000* | 30.9 | 31.6 |
| *2000* | 30.2 | |
| *2000* | 32.0 | |
| *2000* | 31.4 | |
| *2000* | 32.2 | |
| *2000* | 32.7 | |

**Table 4.- Degradability of dry matter and organic matter of diet 1 (100% alfalfa hay) and diet 2 (50% alfalfa hay + 50% concentrate) with different concentrations of additives PTS and PTSO after 48 hours of incubation**

| Diet | Additive | Concentration | DDM, % | DOM, % |
|---|---|---|---|---|
| D1 | | 0 | 56.3 | 57.4 |
| D1 | | 0 | 52.8 | 53.8 |
| D1 | PTS | 50 | 46.2 | 47.5 |
| D1 | PTS | 50 | 46.9 | 45.9 |
| D1 | PTS | 300 | 41.8 | 42.5 |
| D1 | PTS | 300 | 39.4 | 39.5 |
| D1 | PTS | 750 | 39.6 | 38.6 |
| D1 | PTS | 750 | 39.7 | 39.7 |
| D1 | PTS | 1000 | 39.1 | 38.6 |
| D1 | PTS | 1000 | 39.1 | 38.9 |
| D1 | PTS | 2000 | 40.2 | 40.3 |
| D1 | PTS | 2000 | 38.9 | 39.7 |
| D1 | PTSO | 50 | 45.5 | 46.0 |
| D1 | PTSO | 50 | 45.7 | 45.7 |
| D1 | PTSO | 300 | 36.6 | 36.2 |
| D1 | PTSO | 300 | 35.8 | 34.9 |
| D1 | PTSO | 750 | 31.0 | 30.2 |
| D1 | PTSO | 750 | 29.9 | 29.6 |
| D1 | PTSO | 1000 | 33.4 | 32.6 |
| D1 | PTSO | 1000 | 35.6 | 34.7 |
| D1 | PTSO | 2000 | 31.9 | 30.6 |
| D1 | PTSO | 2000 | 28.7 | 27.5 |
| D2 | | 0 | 66.4 | 66.3 |
| D2 | | 0 | 67.2 | 67.4 |
| D2 | PTS | 50 | 60.9 | 60.9 |
| D2 | PTS | 50 | 58.7 | 58.8 |
| D2 | PTS | 300 | 49.3 | 48.6 |
| D2 | PTS | 300 | 47.2 | 47.1 |
| D2 | PTS | 750 | 44.8 | 46.5 |
| D2 | PTS | 750 | 42.2 | 44.6 |
| D2 | PTS | 1000 | 46.1 | 45.6 |
| D2 | PTS | 1000 | 45.1 | 44.7 |
| D2 | PTS | 2000 | 45.7 | 38.9 |
| D2 | PTS | 2000 | 44.9 | 38.5 |
| D2 | PTSO | 50 | 61.1 | 62.2 |
| D2 | PTSO | 50 | 63.9 | 64.8 |
| D2 | PTSO | 300 | 44.4 | 43.7 |
| D2 | PTSO | 300 | 46.0 | 45.6 |
| D2 | PTSO | 750 | 39.9 | 39.0 |
| D2 | PTSO | 750 | 36.3 | 35.8 |
| D2 | PTSO | 1000 | 35.9 | 34.8 |
| D2 | PTSO | 1000 | 38.4 | 37.3 |
| D2 | PTSO | 2000 | 37.1 | 32.7 |
| D2 | PTSO | 2000 | 36.3 | 32.0 |

**Table 5.- Degradation of dry matter and organic matter of the experimental diets incubated for 48 hours with 10 and 25 ppm of compounds PTS and PTSO.**

| ***Diet*** | ***Additive*** | ***Concentration*** | ***DDM, %*** | ***DOM, %*** |
|---|---|---|---|---|
| *D1* | *PTS* | *10* | *54.5* | *54.8* |
| *D1* | *PTS* | *10* | *53.9* | *54.2* |
| *D1* | *PTS* | *25* | *52.5* | *42.9* |
| *D1* | *PTS* | *25* | *52.0* | *43.0* |
| *D1* | *PTSO* | *10* | *54.9* | *45.0* |
| *D1* | *PTSO* | *10* | *56.1* | *51.8* |
| *D1* | *PTSO* | *25* | *54.1* | *52.9* |
| *D1* | *PTSO* | *25* | *55.0* | *53.6* |
| *D2* | *PTS* | *10* | *64.0* | *63.8* |
| *D2* | *PTS* | *10* | *63.9* | *64.0* |
| *D2* | *PTS* | *25* | *57.4* | *57.6* |
| *D2* | *PTS* | *25* | *61.4* | *61.8* |
| *D2* | *PTSO* | *10* | *63.1* | *63.2* |
| *D2* | *PTSO* | *10* | *63.7* | *64.3* |
| *D2* | *PTSO* | *25* | *62.4* | *58.4* |
| *D2* | *PTSO* | *25* | *62.7* | *62. 8* |

**Table 6.- Effect of the addition of different concentrations of compounds PTS and PTSO on the degradability of dry matter (DDM) and organic matter (DOM) of the experimental diets after 48 hours of incubation in ruminal inoculum.**

| | Alfalfa hay | | | | Alfalfa hay + concentrate | | | |
|---|---|---|---|---|---|---|---|---|
| | DDM | | DOM | | DDM | | DOM | |
| Additive, ppm | PTS | PTSO | PTS | PTSO | PST | PTSO | PST | PTSO |
| *0* | 54.5^{a} | 54.5^{a} | 55.6 ^{a} | 55.6^{a} | 66.8 ^{a} | 66.8^{a} | 66.8 ^{a} | 66.8^{a} |
| *10* | 54.2^{a} | 55.5^{a} | 54.5 ^{a} | 48.4^{a b} | 63.9 ^{ab} | 63.4^{a} | 63.9 ^{ab} | 63.8^{a} |
| *25* | 52.3^{a} | 54.5^{a} | 42.9 ^{bc} | 53.2^{a b} | 59.4 ^{b} | 62.5^{a} | 59.7 ^{b} | 60.6^{a} |
| *50* | 46.6^{b} | 45.6^{b} | 46.7 ^{b} | 45.9^{b} | 59.8 ^{b} | 61.1^{a} | 59.8 ^{b} | 63.5^{a} |
| *300* | 40.6^{c} | 36.2^{c} | 41.0 ^{c} | 35.5^{c} | 48.3 ^{c} | 45.2^{b} | 47.8 ^{c} | 44.7^{b} |
| *750* | 39.6^{c} | 30.4^{d} | 39.2 ^{c} | 29.9^{c} | 43.5 ^{c} | 38.1^{c} | 45.5 ^{c} | 37.4^{c} |
| *1000* | 39.1^{c} | 34.5^{c d} | 38.8 ^{c} | 33.7^{c} | 45.6 ^{c} | 37.2^{c} | 45.1 ^{c} | 36.0^{c} |
| *2000* | 39.6^{c} | 30.3^{d} | 40.0 ^{c} | 29.0^{c} | 45.3 ^{c} | 36.7^{c} | 38.7 ^{d} | 32.3^{c} |
| SEM | 0.802 | 0.992 | 0.91 0 | 1.532 | 1.036 | 0.99 | 0.967 | 1.245 |

Different superscript letters in the same column represent significant differences (P<0.001) for each value due to the effect of the concentration of additive; SEM: standard error of the mean.

### Example 4.

The effect of different doses of the compounds object of study on the production of gas promoted by *in vitro* fermentation for 24 and 48 hours of the diets the composition of which is included in Table 1 is studied.

Ruminal fermentation is an ineffective process in that part of the energy ingested by the animal in its diet is lost in the form of methane.

Methane is generated by the action of archaea present in the rumen.

This gas is considered a "greenhouse effect" gas.

Therefore, the reduction of its production in the rumen involves two positive effects: an economic effect since it would determine a more efficient use of the energy in the diet and an environmental effect (Tedeski et al., 2003; Singh et al., 2005) The results (Table 7) indicate (Table 8):
i) Doses greater than 25-50 ppm of the compounds called PTS and PTSO determine a significant decrease of the amount of gas produced by fermentation both for 24 and for 48 hours, by the fermentation of diet D1.
   However, in the case of diet D2 the inhibitory effect of compounds PTS and PTSO on the production of the fermentation gas is observed with lower doses (10 ppm).
   Compounds PTS and PTSO would be more effective in this sense with type D2 diets.
ii) The inhibitory effect of the compounds (PTS and PTSO) on the production of the fermentation gas is time-dependent.
iii) The inhibitory effect of the compounds (PTS and PTSO) on the production of the fermentation gas is dose-dependent.

Despite the fact that the reduction of the fermentation gas is of enormous interest since it would have economic and environmental benefits, as previously mentioned, the magnitude of this effect should be balanced with the magnitude of the effect on ruminal degradation explained in Example 3.

The most beneficial balance will depend on the interest in different circumstances of animal production.

The inhibitory effect of the studied compounds on the production of gas is most likely exerted through its effect on methane-producing microorganisms.

Having sufficiently described the nature of the invention as well as a manner of putting it into practice, it is not considered necessary to provide further explanation so that a person skilled in the art can understand its scope and the advantages derived from it, stating that within its essential features, such invention could be carried out to practice in other embodiments differing in detail from the embodiment indicated by way of example which would be equally comprised within the protection obtained provided that its essential principle is not altered, changed or modified.

## Claims

1. A use of particular antibacterial compounds, which are derived from alliaceae, as natural additives in animal feed, intended as antimicrobial agents, through their inclusion in the diet, for the prevention and/or treatment of digestive pathological processes without affecting the beneficial microbiota, and as an alternative to the use of antibiotics as growth promoters (with a beneficial effect on the production and health condition of the animals, the quality of their products, and on the environment), **characterized by** being compounds present in plants and in that said compounds specifically are any of the following:
- propyl propylthiosulfinate
- n-butyl n-butylthiosulfinate
- propyl propylthiosulfonate
- a compound consisting of a mixture of E and Z isomers or of each of them separately: (E- and Z-4,5,9,-trithiadodeca-1,6,11-triene-9-oxide)
- 3-vinyl-4-H-1,2-dithiin
- 2-vinyl-4-H-1,3-dithiin.

2. The use of particular antibacterial compounds, which are derived from alliaceae, as natural additives in animal feed according to claim 1, **characterized in that** said compounds can be used separately for their inclusion in the animal diet as the only pure active ingredients (purity greater than 95%), as combinations of several of them or in mixtures with other synergistic products; said compounds also being able to be in encapsulated structures or supported on different inert materials or food coatings forming part of the animal diet.

3. The use of particular antibacterial compounds, which are derived from alliaceae, as natural additives in animal feed according to claims 1 and 2, **characterized in that** the concentrations of the active compounds for their inclusion in the animal diet can vary in the final formulation of the feed between the following dose ranges: 1-5 ppm as minimum dose and 10% (w/w) as maximum dose; depending on the compound and/or support, combination thereof and type of animal diet for which they are intended and the physiological condition of the animal (productive or not), as antimicrobials and animal growth promoters.
